# EUROPEAN PATENT APPLICATION

(11) **EP 1 157 682 A1**
(43) Date of publication of application: **28.11.2001**
(21) Application number: 00111229.1
(22) Date of filing: 25.05.2000
(51) Int. Cl.: A61J 1/03, B65D 75/34

(54) **Blister package for topiramate tablets**

(71) Applicant: Cilag AG, 8205 Schaffhausen (CH)
(72) Inventor: Dobler, Monika, 8200 Schaffhausen (CH); Merkle, Stefan, 78244 Gottmadingen (DE); Lorca, Pedro, 8200 Schaffhausen (CH); Bachmann, Dieter, 8234 Stetten (CH)
(74) Representative: Blum, Rudolf Emil Ernst

(57) **Abstract**

The present invention provides a blister package for topiramate tablets comprising a pan sheet having preformed cavities containing topiramate tablets dried to a free water content of between about 0.4% to about 1.4% and a cover sheet sealed to the pan sheet. The present invention further provides a process for blister packaging dried topiramate tablets into a blister package with improved shelf-life and stability performance.

## Description

### FIELD OF THE INVENTION

The present invention provides a blister package for topiramate tablets. More particularly, the blister package contains dried topiramate tablets and comprises a metal layer cover sheet sealed to a composite metal and plastic layer pan sheet.

### BACKGROUND OF THE INVENTION

The pharmaceutical industry employs a variety of dosage formulations for orally administering medicinal agents to patients. Typical formulations for oral administration include liquid solutions, emulsions, or suspensions, as well as solid forms such as capsules or tablets (as used herein, the term "tablet" means any shaped and compressed solid dosage form, including caplets). Methods for preparing tablets are well known in the art, such that the drug agent contained therein is kept stable and active. Accordingly, tablet quality is measured against specifications such as appearance, hardness and drug agent availability as shown by dissolution rate and content uniformity.

A variety of chlorosulfate and sulfamate esters of 2,3:4,5-bis-O-(1-methylethylidene)-β-D-fructopyranose, their anticonvulsant activity in mammals and, thus, their utility in treating epilepsy are described in U.S. Patent No. 4,513,006. More specifically, the compound 2,3:4,5-bis-O-(1-methylethylidene)-β-D-fructopyranose sulfamate, hereinafter referred to as "topiramate", is presently commercially available as a tablet product in strengths of 25, 50, 100 and 200 mg as adjunctive therapy for the treatment of adults with partial onset seizures (TOPAMAX® (topiramate) Tablets). Topiramate can be prepared following the processes disclosed in U.S. Patent Nos. 4,513,006 and 5,387,700 and, preferably, by the process described in Examples 1 to 3 of U.S. Patent No. 5,387,700. Exposure to moisture and heat, though, causes the active agent of 5,387,700. Exposure to moisture and heat, though, causes the active agent of topiramate in the solid dosage form to degrade. Degradation of topiramate tablets is readily detected by changes in physical appearance (discoloration of tablet color to brown or black) and by the formation of sulfate ions which can be readily detected by standard techniques known to those of ordinary skill in the art (e.g., High Pressure Liquid Chromatography).

To maintain tablet quality, topiramate tablets have been packaged into both high-density polyethylene (HDPE) bottles containing a desiccant and blister packages containing a desiccant. Stability testing over time has demonstrated that the tablets in such packages have been preserved under various temperature, humidity and light conditions. Blister packages, however, offer advantages over the HDPE bottles used in the current marketed package and are, therefore, a preferred packaging format for topiramate tablets. Blister packages match the stability and marketing characteristics of HDPE bottles while being less expensive to package, lighter in weight and more conveniently stored; in addition, they offer rapid access, unit dose accountability and better physical protection for the product. Blister packages are especially advantageous for packaging moisture sensitive tablets such as topiramate because each tablet cavity then becomes a primary container in direct contact with the tablet, inherently enclosing a minimum of air and associated moisture.

Tablet stability in a blister package, therefore, becomes a function of the physical characteristics of the materials used in the composite blister package which affect permeability to moisture vapor and the ability to protect the enclosed product from light and humidity. Although other factors affect tablet stability, such as tablet moisture content and the packaging environment itself, manufacturers and packagers have focused on enhancing the stability performance of blister packages by providing cavities for additional materials such as desiccants in addition to the tablet cavities. For example, EP 0466068 A describes a blister package wherein each tablet cavity is connected with one desiccant cavity. Similarly, FR 2593152 A describes a blister package wherein several tablet cavities are connected with one desiccant cavity. Since the desiccant cavity can be accessed as easily as the tablet in the blister packages described in these references, such packages present a potential safety hazard. Recognizing this, FR 2660634 A describes a blister package wherein access to the desiccant cavity is reduced by providing an additional sheet that must be removed to open the desiccant cavity. U. S. Patent Numbers 3,780,856 and 3,835,995 also each describe blister packages with peelable sections that allow access only to the oral dosage form cavities. Furthermore, EP 0779872 B1 describes an oral dosage form cavity for topiramate tablets connected with one desiccant cavity wherein the desiccant cavity is reinforced and cannot easily be opened. In summary, the foregoing references describe blister packages containing a desiccant and the attempts to preserve consumer safety by preventing access to the desiccant cavity.

An object of the present invention is to provide a blister package for topiramate tablets which preserves the stability of the active agent without a desiccant contained therein. Another object of the present invention is to provide an aluminum-aluminum blister package for topiramate tablets with improved stability and shelf-life. An additional object of the present invention is to provide a process for blister packaging dried topiramate tablets.

### SUMMARY OF THE INVENTION

The present invention provides a blister package for topiramate tablets comprising a pan sheet having preformed cavities containing topiramate tablets, and a cover sheet sealed to the pan sheet; wherein the topiramate tablets have a free water content of between about 0.4% to about 1.4% (preferably, between about 0.7% to about 1.2%, more preferably, between about 0.8% to about 1.0%) at the time the cover sheet is sealed to the pan sheet, and wherein the blister package thus formed contains no desiccant.

The present invention is also directed to a process for making a blister package containing topiramate tablets comprising the steps of:
(a) drying a plurality of topiramate tablets to a free water content of between about 0.4% to about 1.4%; preferably, between about 0.7% to about 1.2%; more preferably, between about 0.8% to about 1.0%;
(b) placing the dried topiramate tablets from step (a) into a pan sheet having a plurality of cavities; and
(c) sealing a cover sheet to the filled pan sheet from step (b) to form the blister package, provided that the dried topiramate tablets have a free water content of less than about 1.4% at the time the cover sheet is sealed to the pan sheet and wherein the blister package contains no desiccant.

In an embodiment of the present invention, the topiramate tablets are dried by a method selected from the group consisting of microwave drying, vacuum drying, hot air drying, infrared drying, drying using very dry air either statically or dynamically and drying by placing a desiccant in a storage drum of bulk product for a period of time.

In a class of the invention, the pan sheet is a sheet selected from the group consisting of a single metal layer, multiple metal layers, a single plastic layer, multiple plastic layers, and a composite metal and plastic layer, and the cover sheet is a sheet selected from the group consisting of a single metal layer, multiple metal layers, a composite metal and plastic layer, a composite metal and paper layer and a composite metal, plastic and paper layer.

The metal sheet layer is aluminum foil. The plastic sheet layer is selected from the group consisting of polyvinylchloride (PVC), polyvinyldichloride (PVDC), polyvinylidenchloride, high density polyethylene (HDPE), low density polyethylene (LDPE), polypropylene (PP), cyclicolefin copolymer (COC), polyamide (PA), ortho-polyamide (OPA), polyester (PE), polyacrylate, polyacrylnitril, polystyrene, nylon, polychlorotrifluoroethylene, polycarbonate, ethylene vinyl alcohol copolymer, polyethylene terephthalate (PET), polyethylene terephthalate glycol (PETG) and amorphous PET (APET). The paper layer is selected from the group consisting of paper and waxed paper; wherein the paper layer has an outermost surface to adhere an additional coating including, but not limited to, ink, sealant, lacquer or paint.

Multiple layers are adhered together into a single sheet using techniques known to those skilled in the art. A composite layer refers to metal, plastic and/or paper layers adhered together using techniques known to those skilled in the art. When the pan sheet or cover sheet comprises multiple or composite layers, the metal, plastic and/or paper layers may be independently selected from one or more of the materials listed above. Examples of a multiple plastic layer include, but are not limited to, PVC/PVDC, PVC/PE/PVDC, PVC/PVDC/PE and PVC/PE. Examples of a composite metal and plastic layer include, but are not limited to PVC/metal, OPA/metal/PVC, PVC/PVDC/metal, PET/metal, PET/PVDC/metal and PET/PE/PVDC/metal, wherein the metal is aluminum foil. An example of a composite metal and paper layer is aluminum foil/paper.

In a sub-class of the invention, the pan sheet is a composite metal and plastic layer and the cover sheet is a metal layer.

Illustrative of the invention is the blister package wherein the pan sheet is a composite metal and plastic layer of OPA/aluminum foil/PVC and the cover sheet is a single aluminum foil layer.

### BRIEF DESCRIPTION OF THE DRAWINGS

Figure 1 shows the stability results over time for 100 mg and 200 mg topiramate tablets having various amounts of free water stored at 40°C/75% Relative Humidity (RH).
Figure 2 shows the stability results over time for 100 mg and 200 mg topiramate tablets having various amounts of free water stored at 30°C/60% RH.
Figure 3 shows the rate of moisture re-uptake by dried 25 mg topiramate tablets under various humidity conditions.

### DETAILED DESCRIPTION OF THE INVENTION

In general, the process for preparing topiramate tablet blister packages includes the steps of:
a) obtaining commercially available topiramate tablets;
b) drying the tablets; and,
c) packaging the dried topiramate tablets into blister packages without a desiccant.

The drying methods include, without limitation, microwave drying, vacuum drying, hot air drying, infrared drying, using very dry, heated air either statically or dynamically or placing a desiccant in a storage drum of bulk tablets for a period of time. As a result of using these methods to dry the tablets, the temperature of the surrounding air may vary over a broad range, but should not be so high as to render the active agent inactive.

One skilled in the art can use the drying methods of this invention to achieve optimum tablet drying rates regardless of batch size. In particular, this invention contemplates using dynamic hot air drying, increasing the inlet air volume in a tablet coating drum by 10 m³ per Kg of tablets from 850 m³ for a tablet batch size of 42 Kg to 2800 m³ for a tablet batch size of 240 Kg and varying the drum speed from interval rotation to full rotation based on the tablet batch size. The term "interval rotation" refers to the practice of rotating a coating drum at a standard coating drum rotation speed in revolutions per minute (rpm) for less than one minute. For example, an interval rotation of "10s/min at 10 rpm" would describe rotating a coating drum at a speed of 10 rpm for 10 seconds and then allowing the drum to coast to idle for 50 seconds. In contrast to interval rotation, the term "full rotation" refers to the practice of rotating a coating drum at a standard coating drum rotation speed. For example, a full rotation of "3 rpm" would describe rotating a coating drum at a continuous speed of 3 rpm.

The improved tablet stability and blister package shelf-life contemplated by the present invention depends on the unbound water, hereinafter referred to as "free water," content of the tablets after further drying. Since the total water content of the tablets, hereinafter referred to as "total water," as determined by Karl-Fischer (KF) titration, includes both crystalline bound water, hereinafter referred to as "bound water," and free water, the free water content can be calculated by subtracting the bound water content from the total water content. The bound water is a constant that can be calculated from the known amount of lactose in the granulate formulation.

Accordingly, as shown in Table 1, topiramate tablet formulations with different amounts of lactose will have different amounts of bound water existing within the tablets. The target free water content contemplated by the present invention for such formulations is between about 0.4% to about 1.4% free water; preferably, from about 0.7% to about 1.2% free water; more preferably, from about 0.8% to about 1.0% free water; and, most preferably, less than about 1.0% free water.

**Table 1**

| **Calculation of Free Water Content** | | |
|---|---|---|
| | **25 mg, 50 mg, 100 mg tablets (%)** | **200mg tablets (%)** |
| Target free water | <1.00% | <1.00% |
| Target total water | <2.86% | <1.60% |
| Lactose monohydrate | 37.00% | 11.99% |
| Calculated bound water | 1.86% | 0.60% |
| Calculated free water | <1.00% | <1.00% |

As used herein, the term "blister package" encompasses any type of layered package comprising a product contained between sheets of material; wherein the sheets are adhered or sealed together by methods known to those skilled in the art; for example, using an adhesive activated by heat and/or pressure. The sheets of material are commercially available as either individual sheets (for hand packaging) or, preferably, as a continuous websheet on roll stock (for machine packaging). In addition, the sheets are available in a variety of thicknesses.

The present invention contemplates the use of a pan and cover sheet with moisture vapor barrier properties which limit moisture uptake per tablet to less than 0.1 mg/month under accelerated storage conditions (40°C/75%RH) and includes those aforementioned materials, without limitation, comprising single-layer, multiple-layer or composite layers.

The term "appropriate moisture vapor barrier properties" is intended to encompass accepted moisture vapor permeability criteria known to those skilled in the art for single-unit containers and unit-dose containers for capsules and tablets. In particular, the invention contemplates the use of pan and cover sheets which limit moisture uptake per tablet to less than 0.1 mg/month under accelerated storage conditions (40°C/75%RH).

The "pan sheet" of a blister package refers to the bottom sheet of the package and is made of materials as previously described. In addition, the pan sheet may have a plurality of cavities preformed therein for the retention of the topiramate tablets being packaged. During the packaging process, the pan sheet cavities are formed and filled with tablets. The pan sheet is then covered and sealed with a top sheet, hereinafter referred to as the "cover sheet." The cover sheet is made of materials as previously described. In the present invention, for example, the blister package may comprise a composite metal and plastic layer OPA/alu/PVC pan sheet and a cover sheet having a single aluminum foil layer, hereinafter referred to as an "alu-alu" blister package.

Alternatively, the pan sheet and the cover sheet may each be independently formed from continuous sheets of roll stock registered for being simultaneously fed as websheets on a blister packaging machine. As either a single sheet or as a websheet, the cover sheet may further comprise a composite metal and paper layer; wherein the paper layer has a surface capable of accepting print. Preferably, the metal layer is aluminum foil having a paper layer adhered thereto; wherein the paper layer is commercially available in a variety of thicknesses and is adhered to the metal layer using methods known in the art.

The following specific examples are presented by way of illustration and are not intended to limit the scope of the claims of the present invention.

### Example 1

### Pilot Scale Drying Process

Several 42 kg tablet batches formulated at different strengths were independently placed in a GLATT GC 750/500 (59-I-drum) coater, manufactured by GLATT AG, Pratteln, Switzerland. The coater drum was rotated at intervals of 10 seconds per minute at a speed of 10 rpm; for the remaining 50 seconds per minute, the drum was not rotated; the incoming air was heated to about 70°C. The rate of tablet drying was controlled by the process parameters of the coater as determined by measurement of inlet/outlet temperature and air volume. The amount of incoming air was maintained at a rate of between about 800 m³/h to about 950 m³/h and was typically about 900 m³/h to maintain a constant negative pressure in the drum. The free water content for each formulation was determined throughout the drying process by the use of in-process control (IPC) testing. IPC samples were taken every 30 min and tested for loss on drying (LOD) using a Mettler HR73 halogen dryer, manufactured by Mettler-Toledo, Greifensee, Switzerland, wherein 5 gm samples of topiramate tablets were powderized and measured using the dryer's fast dry mode at 105°C with a medium weight loss smaller than about 1 mg in about 50 seconds. The drying time required for each batch of tablets to reach the target free water content (including a period of heating-up the incoming air) varied from about 1.25 h to about 4 h; preferably, from about 2 to about 3 h.

Different drying techniques for the 25 mg, 50 mg, 100 mg and 200 mg tablet strengths were tested and compared. The use of microwave drying, vacuum drying and, preferably, drying in a coater pan with hot air yielded tablets with unchanged product quality specifications including appearance, assay, degradation products and dissolution profile.

The IPC results for the free water content of each pilot scale batch dried according to the process of Example 1 are shown in Table 2.

**Table 2**

| **Pilot Scale Results** | | | | | |
|---|---|---|---|---|---|
| **Strength** | **Drying Time (h)** | **LOD Start (%)** | ^{**1**}**LOD End (%)** | ^{**2**}**Initial KF (%)** | ^{**3**}**Initial Free Water (%)** |
| 25 mg | 2.5 | 2.11 | 1.09 | 2.72 | 0.86 |
| 25 mg | 2.25 | 1.74 | 1.26 | 2.74 | 0.88 |
| 25 mg | 1.25 | 1.74 | 1.43 | 2.96 | 1.10 |
| 25 mg | 3.5 | 1.74 | 1.18 | 2.67 | 0.81 |
| 50 mg | 2.5 | 2.10 | 1.11 | 2.76 | 0.90 |
| 100 mg | 2.5 | 1.98 | 1.09 | 2.66 | 0.80 |
| 200 mg | 3 | 1.54 | 0.84 | 1.41 | 0.80 |
| 200 mg | 2 | 1.98 | 1.19 | 1.61 | 1.00 |
| 200 mg | 4 | 1.98 | 0.93 | 1.40 | 0.79 |
| 200 mg | 3 | 1.98 | 1.07 | 1.48 | 0.87 |

| | | | | | |
|---|---|---|---|---|---|
| ¹ Sampled at the end of the drying process | | | | | |
| ² Initial Total Water (KF): After packaging into alu/alu blisters | | | | | |
| ³ Initial Free Water calculated by subtracting the crystalline bound water from KF (i.e., 1.86% for the 25 mg, 50 mg and 100 mg tablets and 0.60% for the 200 mg tablets) | | | | | |

Similar LOD results for production scale tablet batches were achieved using comparable equipment for scale-up from pilot batch manufacture as shown in Table 3.

**Table 3**

| **Production Scale Equipment** | | |
|---|---|---|
| | **Pilot Scale** | **Production scale** |
| Batch Size (approximate) | 42 Kg | 240 Kg |
| GLATT Coater | GC750/500 | GC1350 |
| Inlet Air Volume | 850 m³ | 2800 m³ |
| Inlet Air Moisture | Not Controlled | 10 g/Kg |
| Inlet Air Temperature | 70°C | 70°C |
| Drum Revolutions | 10s/min at 10 rpm | 3 rpm |
| Optimum Drying Time | 2.5 h | 2.5 h |

For this Example, drying time was the same for both pilot and production scale drying using the method of the present invention.

### Example 2

### Blister Packaging Topiramate Tablets

The topiramate tablets dried by the procedure of Example 1 were blister packaged into an alu/alu blister package in accordance with standard operating procedures, conforming to current good manufacturing practices and using packaging materials known to one of ordinary skill in the art:

### Blister Packaging Procedure

1. A blister packaging machine forming die design 1PJ-521-030 and 1PJ-521-020 prepared by Uhlmann Pac-Systeme GmbH & Co KG, Laupheim, Germany was used on a Harro Höfliger Type PFM65 or PFM77 blister packaging machine manufactured by Harro Höfliger Verpackungsmaschinen GmbH, Allmersbach, Germany; the forming die design had a side by side put-up of four 53 mm x 130 mm blister packages per pan sheet width, each blister package having 10 single-tablet cavities perforated for single-unit dosing.
2. The blister package pan sheet was prepared from a continuous sheet of OPA/alu/PVC 25 µm/45 µm/60 µm foil on roll stock manufactured by Lawson Mardon Neher AG, Kreuzlingen, Switzerland was web-fed at 18 cycles per minute to the pan sheet forming die wherein the tablet cavities were cold-formed by the forming punches.
3. The pan sheets thus formed were web-fed as a continuous sheet to an automatic tablet feeder which contained a batch of topiramate tablets further dried according to the procedure of Example 1. The batch size in the tablet feeder may range from about 42 Kg for a pilot scale batch of approximately 100,000 200 mg tablets to about 240 Kg for a production scale batch of approximately 3,000,000 25 mg tablets or sublots thereof. Alternatively, tablets may be manually fed into the preformed tablet cavities. The invention contemplates the use of both automatic and manual tablet feeding.
4. The blister package cover sheet was prepared from a continuous 20.0 µm (54.0 g/m²) sheet of hard aluminum foil with a heatseal lacquer (7.0 g/m²) LA723 on roll stock manufactured by Lawson Mardon Neher AG, Kreuzlingen, Switzerland simultaneously web-fed at 18 cycles per minute with the pan sheet web to a sealing die maintained at a temperature of about 180°C to about 190°C, wherein the web-fed cover sheet was sealed to the web-fed pan sheet at a sealing pressure of 80 bar, to thus simultaneously form a set of four side by side sealed blister packages.
5. The set of four sealed blister packages was then web-fed to a cutting die, whereby the cutting die simultaneously perforated the sealed area around the filled tablet cavities to form single tablet dosage-units and cutting into single 10 tablet dose-unit blister packages.

### Packaging Room Conditions

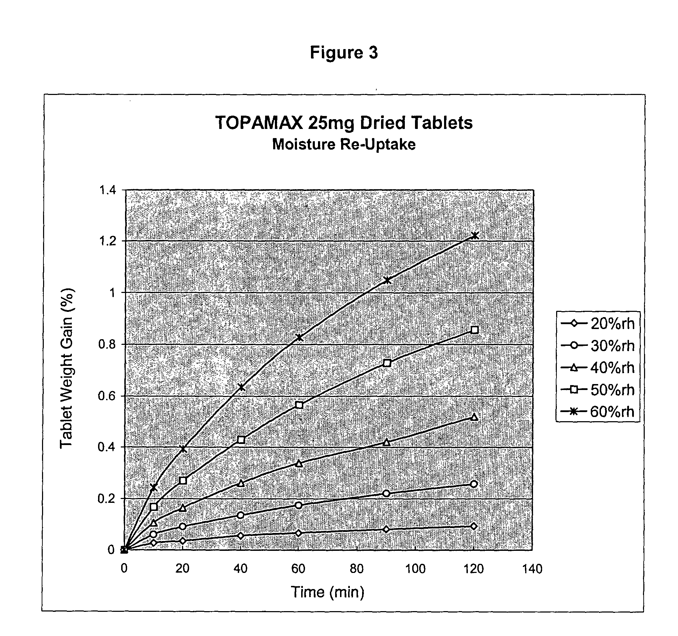

As shown in Figure 3, the re-uptake of moisture by dried 25 mg topiramate tablets depends on the relative humidity of the packaging room air. In an embodiment of this invention, air moisture is preferably maintained at less than 20% RH.

### Example 3

### Blister Package Evaluation Study

Against the appearance specifications defined in Table 5 below, a package evaluation study was conducted wherein appearance data was generated for commercially available topiramate tablets having a free water content of about 1.7% that were packaged into various package formats:
1. DUMA HDPE bottles (30, 50, 75 ml), manufactured by DUMA International AB, Stockholm, Sweden, having a desiccant according to approved marketed specifications independently containing 60 tablets of the 25, 50, 100 or 200 mg strengths used as a reference;
2. Alu/alu blister packages having an OPA/Alu/PVC (25µm/45µm/60µm) pan sheet and a hard aluminum foil (20µm) cover sheet and 10 cavities each containing a tablet of the 25, 50, 100, or 200 mg strength;
3. Blister packages having a PVC/PE/PVDC (200µm/25µm/90g/m²) (sold under the Tristar® brand of plastic film) pan sheet and a hard aluminum foil (20µm) cover sheet and 10 cavities each containing a tablet of the 25, 50, 100, or 200 mg strength were placed in a PE/Alu/PE (12µm/12µm/75µm) pouch with a Minipax™ (2 g Silicagel) desiccant unit therein, hereinafter referred to as the "blister in pouch" package;
4. Blister packages having a PVC/PE/PVDC (200µm/25µm/90g/m²) (sold under the Tristar® brand of plastic film) pan sheet and a hard aluminum foil (20µm) cover sheet and 10 cavities each containing a tablet of the 25, 50, 100, or 200 mg strength and each connected with one of 10 cavities each containing a desiccant tablet (0.5 g Silicagel);
5. Blister packages having a PVC/PE/PVDC (200µm/25µm/90g/m²) (sold under the Tristar® brand of plastic film) pan sheet and a hard aluminum foil (20µm) cover sheet and 10 cavities each containing a tablet of the 25, 50, 100, or 200 mg strength.

The plastic film used for the foregoing blister packages was manufactured by PERLEN CONVERTING AG, Perlen, Switzerland and marketed under the trademark Tristar® (brand of plastic film). According to the schedule shown below in Table 4, the packages described above were placed into stability chambers under various conditions and periodically tested. At the times and under the conditions shown in Table 4, (X) indicates that testing was performed on request only. At the times and under the conditions shown in Table 4, X indicates that appearance, active ingredient and sulfate assay, purity and dissolution testing were performed. Appearance testing was performed every month during the first 7 months and as indicated by X in Table 4.

**Table 4**

| Blister Evaluation Study: | | | | |
|---|---|---|---|---|
| **Storage time (months)** | **5°C** | **25°C/60%RH** | **30°C/60%RH** | **40°C/75%RH** |
| 0 | -- | X | -- | -- |
| 3 | -- | -- | -- | -- |
| 6 | -- | X | -- | X |
| 9 | -- | X | X | |
| 12 | (X) | X | X | |
| 18 | -- | X | X | |
| 24 | (X) | X | X | |
| 36 | (X) | X | X | |
| 48 | (X) | X | X | |

The study results for each package type are shown below in Table 5.

The study demonstrated that the appearance of topiramate tablets with a free water content of about 1.7% for:

### Package Format 1

1. Continued to pass the appearance specification at room temperature (RT) after 48 months.
2. Passed the appearance specification under 30°C/60% RH conditions at 36 months but would have failed the 48 month time point.
3. Passed the appearance specification under 40°C/75% RH conditions at 6 months.

### "Alu/Alu" Blister Package Format 2

1. Continued to pass the appearance specification at RT after 48 months.
2. Failed the appearance specification under 30°C/60% RH conditions at 24 months (product standards require passing the appearance specification at 36 months).
3. Failed the appearance specification under 40°C/75% RH conditions at 5 months (product standards require passing the appearance specification at 6 months).

### "Blister In Pouch" Package Format 3

1. Continued to pass the appearance specification at RT after 48 months.
2. Continued to pass the appearance specification under 30°C/60% RH conditions after 48 months.
3. Continued to pass the appearance specification under 40°C/75% RH conditions after 36 months.

### Package Format 4

Study was stopped after 6 months for all conditions (package format too expensive for marketing).

### Package Format 5

1. Study was stopped after 12 months at RT and 30°C/60% RH conditions
2. Failed the appearance specification under 40°C/75% RH conditions after 3 months (product standards require passing the appearance specification at 6 months).

Package format 1 provided protection that met product specifications. but a pharmaceutically elegant, less expensive alternative package is desired. The "blister in pouch" package provided better protection from humidity than the other package formats tested, but such a package is bulky and expensive to produce on a commercial scale. The "alu-alu" blister package as studied in Example 3 (i.e., tablets having about 1.7% free water content) did not provide protection that met product specifications. The results of Example 4 for the "alu-alu" blister package, however, demonstrate that by drying the tablet prior to packaging, a surprisingly improved shelf life and stability for topiramate tablets in an "alu-alu" blister package is acheived.

**Table 5**

| **First Visible Change in Tablet Appearance for Example 3 Package Evaluation Study** | | | |
|---|---|---|---|
| **Format** | **RT** | **30°C/60%RH** | **40°C/75% RH** |
| **1** | >48 M | 36 M | 6 M |
| **2** | >48 M | 24 M | 5 M |
| **3** | >48 M | >48 M | 36 M |
| 4 | >6M | >6M | 6M |
| 5 | >12M M | >12M | 3M |

### Appearance Specification

The approved specifications for the stability of topiramate are the appearance specifications as shown in Table 6. The appearance of the tablet has been established as a very sensitive indicator for topiramate stability.

When the first beige specks were visible (code 2), the tablet still met all appearance specifications. If the sulfate level, however, exceeded the specification level of ≤ 0.5 Mole%, the beige specks soon became visibly darker (code 3) and the tablet was considered to have failed the appearance specifications. In accordance with International Committee on Harmonization (ICH) Guidelines, tablets must be maintained within approved specifications under accelerated stability conditions of 40°C/75% RH for at least six months.

**Table 6**

| **Classification Scale for Physical Testing/Appearance** | | | |
|---|---|---|---|
| **Specification** | | **Criteria** | **Pass/Fail** |
| **Code 1** | No Change | White fractures with no visible discoloration | Pass |
| **Code 2** | Very Slight Change | White fracture with no visible discoloration and occasional beige specks | Pass |
| **Code 3** | Definite Change | Discoloured fracture with dark specks | Fail |
| **Code 4** | Change First Noticeable to Consumer | Shade change to the outside of the tablet; acetone-like odor; dark fracture | Fail |
| **Code 5** | Extreme Change | Dark shade change to the outside of the tablet | Fail |

### Example 4

### Alu-alu Blister Package Evaluation Study

Against the appearance specifications defined in Table 6 above, a package evaluation study was conducted under various stability conditions, wherein appearance data was generated for topiramate tablets having a free water content of about 0.8%, 1.4% or 1.8% that were packaged into "alu-alu" blister packages:
1. Alu-alu blister packages having an OPA/Alu/PVC (25µm/45µm/60µm) pan sheet (composite sheet manufactured by Louis Patz&Comp., Natschbach, Austria) and a hard aluminum foil (20µm) cover sheet (manufactured by Tscheulin-Rothal GmbH, Teningen, Germany) and 10 cavities each containing a tablet of the 100 or 200 mg strength, each tablet having a free water content of about 0.8%.
2. Alu-alu blister packages having an OPA/Alu/PVC (25µm/45µm/60µm) pan sheet (composite sheet manufactured by Louis Patz&Comp., Natschbach, Austria) and a hard aluminum foil (20µm) cover sheet (manufactured by Tscheulin-Rothal GmbH, Teningen, Germany) and 10 cavities each containing a tablet of the 100 or 200 mg strength, each tablet having a free water content of about 1.4%.
3. Alu-alu blister packages having an OPA/Alu/PVC (25µm/45µm/60µm) pan sheet (composite sheet manufactured by Louis Patz&Comp., Natschbach, Austria) and a hard aluminum foil (20µm) cover sheet (manufactured by Tscheulin-Rothal GmbH, Teningen, Germany) and 10 cavities each containing a tablet of the 100 or 200 mg strength, each tablet having a free water content of about 1.8%.

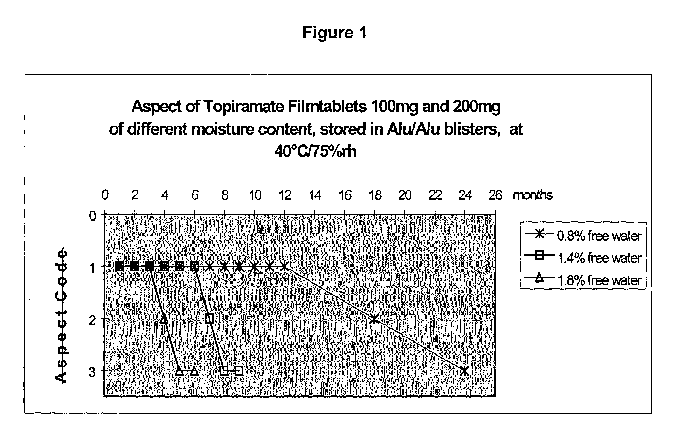

Under accelerated stability conditions of 40°C/75% RH, as shown in Figure 1, the tablet batch with a free water content of 1.8% failed the appearance specification listed in Table 6 after 5 months. The batch with a free water content of 1.4% failed the appearance specification after 8 months. The batch with a free water content of 0.8%, however, surprisingly continued to pass the appearance specification after 12 months and failed only after 24 months.

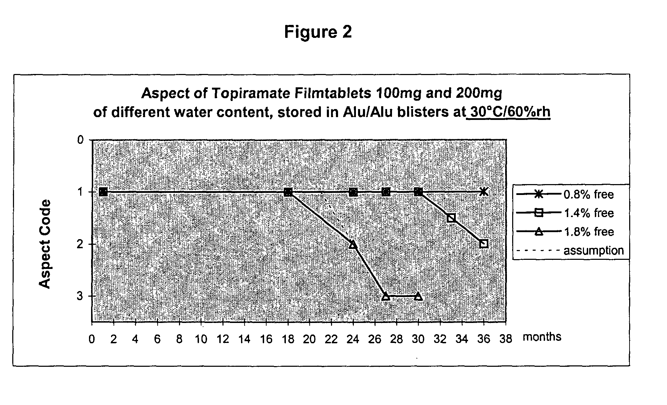

Under stability conditions of 30°C/60% RH, as shown in Figure 2, the batch with a free water content of about 1.8% failed the appearance specification after 24 months; yet, the batches with a free water content of 0.8% free water and 1.4% free water both continued to pass the appearance specification after 36 months.

As also shown in Figure 1, the appearance data resulting from the study demonstrated that the topiramate tablets with a free water content of about 0.8% in an alu/alu blister package met the appearance specification for 18 months at 40°C/75% RH. The study also demonstrated that the topiramate tablets with a free water content of from about 0.8% to about 1.4% in an alu/alu blister package met the appearance specification for 36 months at 30°C/60% RH.

The results of the study after 36 months described in Example 4 are summarized in Table 7.

**Table 7**

| **Change in Tablet Appearance for Example 4** | | |
|---|---|---|
| **Water Content** | **30°C/60%RH** | **40°C/75% RH** |
| 0.8% | Passed 36^{th} Month | Failed 24^{th} Month |
| 1.4% | Passed 36^{th} Month | Failed 8^{th} Month |
| 1.8% | Passed 24^{th} Month | Failed 5^{th} Month |

Comparing the "alu-alu" blister package studied in Example 3 with the results of Example 4 demonstrate that the tablet drying method of the present invention provides a surprisingly improved shelf life and stability for topiramate tablets in the "alu-alu" blister package.

While the foregoing specification teaches the principles of the present invention, with examples provided for the purpose of illustration, it will be understood that the practice of the invention encompasses all of the usual variations, adaptations and modifications for tablet drying and blister packaging known to one skilled in the art that could come within the scope of the following claims and their equivalents.

## Claims

1. A blister package for topiramate tablets comprising a pan sheet having preformed cavities containing topiramate tablets, and a cover sheet sealed to the pan sheet; wherein the topiramate tablets have a free water content of between about 0.4% to about 1.4% at the time the cover sheet is sealed to the pan sheet and wherein the blister package thus formed contains no desiccant.

2. The blister package of claim 1 or 2 wherein the pan sheet is a sheet selected from the group consisting of a single metal layer, multiple metal layers, a single plastic layer, multiple plastic layers, and a composite metal and plastic layer.

3. The blister package of claim 2 wherein the cover sheet is a sheet selected from the group consisting of a single metal layer, multiple metal layers, a composite metal and plastic layer, a composite metal and paper layer, and a composite metal, plastic and paper layer.

4. The blister package of anyone of claims 1 to 3 wherein the pan sheet is a composite metal and plastic layer and the cover sheet is a metal layer.

5. The blister package of claim 4 wherein the pan sheet is a composite metal and plastic layer of ortho-polyamide/aluminum foil/polyvinylchloride and the cover sheet is a single aluminum foil layer.

6. The blister package of claim 5 wherein the pan sheet is a 25 µm ortho-polyamide/45 µm aluminum foil/60 µm polyvinylchloride sheet and the cover sheet is a 20 µm aluminum foil sheet.

7. The blister package of anyone of claims 1 to 6 wherein the topiramate tablets have between about 0.7% to about 1.2% free water.

8. The blister package of claim 7 wherein the topiramate tablets have between about 0.8% to about 1.0% free water.

9. The blister package of anyone of claims 1 to 8 wherein the topiramate tablets are dried by a method selected from the group consisting of microwave drying, vacuum drying, hot air drying, infrared drying, drying using very dry air either statically or dynamically, and drying by placing a desiccant in a storage drum of bulk product for a period of time.

10. The blister package of claim 9 wherein the topiramate tablets are dried using hot air.

11. A process for making a blister package containing topiramate tablets comprising the steps of:
(a) drying a plurality of topiramate tablets to a free water content of between about 0.4% to about 1.4%;
(b) placing the dried topiramate tablets from step (a) into a pan sheet having a plurality of cavities; and
(c) sealing a cover sheet to the pan sheet from step (b) to form the blister package, provided that the dried topiramate tablets have a free water content of less than about 1.4% at the time the cover sheet is sealed to the pan sheet and wherein the blister package contains no desiccant.

12. The process of claim 11 wherein the pan sheet is a sheet selected from the group consisting of a single metal layer, multiple metal layers, a single plastic layer, multiple plastic layers and a composite metal and plastic layer.

13. The process of claim 11 or 12 wherein the cover sheet is a sheet selected from the group consisting of a single metal layer, multiple metal layers, a composite metal and plastic layer, a composite metal, and paper layer and a composite metal, plastic and paper layer.

14. The process of anyone of claims 11 to 13, wherein the pan sheet is a composite metal and plastic layer and the cover sheet is a metal layer.

15. The process of claim 14 wherein the pan sheet is a composite metal and plastic layer of ortho-polyamide/aluminum foil/polyvinylchloride and the cover sheet is a single aluminum foil layer.

16. The process of claim 15 wherein the pan sheet is a 25 µm ortho-polyamide/45 µm aluminum foil/60 µm polyvinylchloride sheet and the cover sheet is a 20 µm aluminum foil sheet.

17. The process of anyone of claims 11 to 16 wherein the topiramate tablets are dried to a free water content of between about 0.7% to about 1.2%.

18. The process of claim 17 wherein the topiramate tablets are dried to a free water content of between about 0.8% to about 1.0%.

19. The process of anyone of claims 11 to 18 wherein the topiramate tablets are dried by a method selected from the group consisting of microwave drying, vacuum drying, hot air drying, infrared drying, drying using very dry air either statically or dynamically, and drying by placing a desiccant in a storage drum of bulk product for a period of time.

20. The process of claim 19 wherein the topiramate tablets are dried using hot air in a coater at a temperature from about 60°C to about 70°C.

21. A blister package made by the process of anyone of claims 11 to 20.
